# EUROPEAN PATENT SPECIFICATION

(11) **EP 0 336 324 B1**
(45) Date of publication and mention of the grant of the patent: **22.02.1995**
(21) Application number: 89105735.8
(22) Date of filing: 31.03.1989
(51) Int. Cl.: C07K 1/113, C07K 1/14, C12P 21/02

(54) **Process for treating insoluble fused heterogenic protein**
Prozess zur Behandlung von unlöslichem heterogenem Fusionsprotein
Procédé de traitement de protéines fusionnées, hétérogènes et insolubles

(30) Priority: 08.04.1988 JP 85284/88
(43) Date of publication of application: 11.10.1989
(73) Proprietor: Tosoh Corporation, Shinnanyo-shi Yamaguchi-ken (JP)
(72) Inventor: Yoshikawa, Kazuhide, Yokohama-shi Kanagawa-ken (JP)
(74) Representative: Grünecker, Kinkeldey, Stockmair & Schwanhäusser Anwaltssozietät

(56) References cited:
- EP-A- 0 192 629
- CRITICAL REVIEWS IN BIOCHEMISTRY, vol. 3, May 1975, pages 1-43, AmericanChemical Society; C.N. PACE: "The stability of globular proteins"
- PROTEIN PURIFICATION MICRO TO MACRO, PROCEEDINGS OF A CETUS-UCLA SYMPOSIUM,Frisco, Colorado, 29th March - 4th April 1987, pages 279-305, Editor R.
- Burgess, Alan R. Liss, Inc., New York, US; M.W. KNUTH et al.: "Purification ofproteins in the denatured state"
- PROTEIN PURIFICATION MICRO TO MACRO, PROCEEDINGS OF A CETUS-UCLA SYMPOSIUM,Frisco, Colorado, 29th March - 4th April 1987, pages 429-442, Editor R.Burgess, Alan R. Liss, Inc., New York, US; P.A. LOWE et al.: "Solubilisation,refolding and purification of eukaryotic proteins expressed in E. coli"
- SPRINGER ADVANCED TEXTS IN CHEMISTRY, 1987, second edition, chapter 3.6, pages64-69, Springer Verlag, New York, US; R.K. SCOPES: "Protein purification:Principles and practice"
- Protein Folding, EPO Applied Technology Series, Vol. 12, p. 1-3, 9-11, 84, 85 (1983)

## Description

### Background of the Invention:

### Field of the Invention:

The present invention relates to a process for solubilization, purification and refolding of a fusion protein consisting of human nerve growth factor and a part of human growth hormone.

### Description of the Related Art:

By means of the gene manipulation technique it is possible to produce protein heterogenic to host cells by transducing into the host cells genes encoding the protein. The heterogenic proteins thus produced often assume a higher order structure which inhibits expression of their own physiological activity and remain in the form of insoluble agglomerates in the host cells. These insoluble heterogenic proteins inevitably require for their effective utilization a series of treatments including solubilization, purification and refolding.

The series of treatments to solubilize and refold insoluble heterogenic protein produced in host cells can be performed by a known art in which, for example, the insoluble heterogenic protein is treated for solubilization in a solution of a strong protein denaturant such as a 4 - 9 M solution of guanidine hydrochloride and then refolded by reducing the concentration of the protein denaturant. In this process, however, the problem is how to handle a large amount of materials, because dialysis or dilution of the solution is required to lower the concentration of the protein denaturant. When such compound as guanidine hydrochloride is used, an additional problem arises in treatment of the exhaust liquid produced. Guanidine hydrochloride cannot be disposed of by a usual method for waste liquid disposal such as the active sludge method.

In "Protein Purification:Micro to Macro", p. 279-305, (1987), it is disclosed that effects of denaturing agents are often additive or synergistic. This is exemplified by the observation that the pH at which a protein denatures is lowered upon addition of chaotropic agents.

Another process is known for the refolding, in which insoluble heterogenic protein is treated for solubilization in an alkali solution and refolded by then decreasing the pH of the solution. In this process, a simpler and easier method is provided to handle a large amount of materials as compared with the preceding process, since the lowering of the pH can be achieved by adding an acid substance.

However, the problem in this method is that the rate of solubilization of insoluble heterogenic protein tends toward a lower value, because the alkali solution is hardly brought into contact with the inside of the agglomerates of said insoluble heterogenic protein. A low rate of solubilization is not preferable because it causes a lower yield.

According to "Protein Purification:Micro to Macro", p. 429-442 (1987), the concentration of the protein is of particular importance, since the optimum concentration may be low (1mg/ml or less) with higher concentrations favouring aggregation or precipitation.

Protein is known to assume the primary, secondary, tertiary and sometimes quaternary structures. The primary structures are composed of about 20 kinds of amino acids and more than 200 different primary structures have so far been established. The secondary and tertiary structures, so-called the higher order structures, are composed by the intramolecular linkage between amino acids in the primary structure of protein. There are known at present more than 100 kinds of established tertiary structures as a result of the X-ray diffraction for crystal structure analysis. Further, the recent advanced gene manipulation technique teaches that even those heterogenic protein which is difficult to be produced in host cells can be produced in a fused form with another protein which is easily produced. In this technique, two or more kinds of protein are produced in a fused form combined via an amino acid sequence such as thrombin, factor Xa, and trypsin which is specifically digested by an arbitrary enzyme, and the product is later treated with said enzyme to produce a protein aimed at.

The proteins or fused proteins thus produced by the gene manipulation technique having various different structures may be supposed to have an optimum condition on the treatments such as solubilization, purification and refolding and so on. For example, an insoluble fused heterogenic protein consisting of human nerve growth factor (hereinafter designated as NGF) and a part of human growth hormone (hereinafter designated as hGH), both produced by Escherichia coli, may be solubilized by treating with an alkali solution, but produce a precipitate when the pH is lowered for the refolding treatment, with a remarkable loss in the yield.

The present inventors have succeeded in solving the problems in prior techniques. Through extensive and continued investigations on the process of treatments such as solubilization, purification and refolding of insoluble fused heterogenic protein so far unknown, and finally completed this invention.

### Summary of the Invention:

The present invention is to provide a process for solubilizing, purifying and refolding a fusion protein consisting of human nerve growth factor and a part of human growth hormone comprising the following steps: a) Bringing said fusion protein into contact with an aqueous acid solution comprising 7 M urea, b) Adjusting the pH to 10 to 13 by adding an alkaline substance and lowering the urea concentration to about 3.5 M, and c) Lowering the pH.

### Detailed Description of the Invention:

The present invention will be described hereinbelow in more details.

The process according to the present invention comprises three steps, corresponding to solubilization, purification and refolding of the protein.

### (1) The solubilization treatment of the insoluble fused heterogenic protein

The present invention provides a process in which broken pieces of bacteria containing insoluble fused heterogenic protein prepared by breaking host cells into pieces, for example, by homogenizing or ultrasonics, or concentrates of the bacteria pieces prepared from the above bacteria pieces by centrifugation, ultrafiltration, or gel filtration are brought into contact with an aqueous acid solution in the presence of a weak denaturant for protein, in order to solubilize the desired fusion protein.

The fused protein is the aimed-at protein which is combined, at the upstream end of the N-terminal, with the whole or a part of an allogenic or heterogenic protein easily producible by host cells used. According to the present invention, the fused protein is composed of NGF and hGH.

Protein denaturants include commonly known denaturants such as, for example, guanidine hydrochloride, urea, surfactants, and thiocyanic acid salts. According to the present invention, urea is the most preferred since it gives least influence to living body and is easy to be disposed of. Concentration of the protein denaturant may be that at which an insoluble fused heterogenic protein is readily dispersed in the solution. That concentration necessary for the dispersion may be appropriately decided mainly considering the nature of the denaturant. When urea is used as denaturant, necessary consideration is that protein is generally denatured by urea at a concentration higher than 2 M and that agglomerates of insoluble fused heterogenic protein may be dispersed at a concentration of urea lower than that at which protein is denatured.

Protein denaturants may be kept either in the form of being dissolved in an aqueous acid solution or copresent with said fused protein before an aqueous acid solution is brought into contact with the agglomerates of insoluble fused protein. The latter seems better because a less amount of the protein denaturant may suffice. In this invention, dispersing an insoluble fused heterogenic protein with a protein denaturant makes it easier for an aqueous acid solution to sink deep into the depth of agglomerate of said fused protein, to improve the rate of solubilization. During the dispersing and solubilizing operations, the whole mixture may be agitated in addition.

The aqueous acid solutions may be aqueous solution of popular acid substances including, for instance, inorganic acids such as hydrochloric acid, thiocyanic acid, hydroiodic acid, and hydrobromic acid, and organic acid such as acetic acid. Among them, however, aqueous solutions of organic acids such as acetic acid are preferred, because organic acids are more effective in the denaturation of fused heterogenic protein. Concentration of the acid substances may be that at which pH of said aqueous solution the fused heterogenic protein aimed at is reversibly acid-denatured. For example, for the fused protein composed of NGF and hGH, a pH of 2 - 5, preferably 3 - 4, should be brought about with an acid substance.

### (2) The purification treatment of insoluble fused heterogenic protein

The step of purifying insoluble fused heterogenic protein according to the present invention provides comprises increasing the pH of the acid solution containing solubilized fused heterogenic protein by adding an alkali substance.

Alkali substances employed may include, for example, sodium hydroxide, potassium hydroxide, sodium carbonate, ammonia, alkylamines and the like without any restriction.

The pH should be elevated to a value where the fused heterogenic protein is reversibly denatured by alkali, and therefore preferably to as high a pH as possible. At a high value of pH, more impurities other than the fused heterogenic protein may precipitate, leading to an improved purification, and at the same time the rate of solubilized protein in an aqueous alkali solution may be improved. In the case of a fused protein composed of NGF and hGH, for example, the pH should be elevated to 10 - 13.

Contaminants which precipitate at a high pH can be removed in any familiar way such as by centrifugation, ultrafiltration, gel filtration and the like.

### (3) The refolding treatment of insoluble fused heterogenic protein

The refolding treatment of insoluble fused heterogenic protein according to the present invention provides consists in lowering the pH of an aqueous alkali solution which contains fused heterogenic protein dissolved in it, in order to refold said protein into a form which is capable of expressing physiological activity.

When a high concentration of protein denaturant is used in the solubilization treatment, however, an additional treatment of lowering the concentration of the denaturant is eventually needed even for the enzymatic digestion of fused protein. Therefore, in the solubilization treatment for refolding, it is preferred for dispersing agglomerates of insoluble fused heterogenic protein either to use protein denaturant of a concentration at which said protein is not denatured, or alternatively to use a denaturant of a high concentration which is expected to be lowered to a value not sufficient to denature said protein when said solution is mixed with an aqueous acid solution. When a high concentration of a protein denaturant is used and the resultant solution after the refolding treatment contains the denaturant of a concentration high enough to denature the fused protein, the concentration may be lowered if necessary by dialysis or dilution. Thus, use of a protein denaturant in the solubilization treatment is advantageous to obtain a higher rate of solubilizing insoluble fused heterogenic protein and therefore to improve the final yield of the protein.

As has been mentioned above, a fused heterogenic protein is first solubilized in an aqueous acid solution and subsequently pH of said solution is elevated as explained in the treatment (2) above.

As an alkali, familiar alkali substances may be employed as described above in the purification treatment. But organic bases such as ammonia, triethylamine are preferred since they promise a higher rate of refolding than other alkali substances do. Additionally, a further higher rate of refolding can be expected when an aqueous solution of at least one organic base is employed which is selected from the group consisting of monoethanolamine, diethylenetriamine, triethylenetetramine, and tetraethylenepentamine. The reason an aqueous solution of an organic base effectively brings about refolding of fused heterogenic protein at a high rate is not clear. Further among organic bases, monoethanolamine, diethylenetriamine, triethylenetetramine, and tetraethylenepentamine cause to refold at a higher rate. The reason for it is also not clear, but presumably the straight chain structure of these compounds may have any contribution to the fact.

As has been explained for treatment (2), the pH should be elevated to a range in which the fused protein is reversibly denatured by alkali. For a fused protein composed of NGF and hGH; a pH less than 10 is insufficient for the denaturation in an alkaline solution and a pH greater than 13 is apt to lead to irreversible denaturation. Thus a pH in the range 10 - 13 is preferable. A pH in the range 11 - 12 is more preferable to obtain a higher rate of refolding. Presumably, this is because the pH delicately influences refolding as well as the solubilization in an alkaline medium.

By the operations described above, an alkaline solution which stably contains the fused heterogenic protein dissolved can be obtained. The fused heterogenic protein composed of NGF and hGH, if only solubilized with an aqueous alkaline solution, may be precipitated when the pH of the solution is lowered for refolding, but by the operations described above it remains in the solution without being precipitated, and furthermore impurities can be removed. The removal of impurities may be conducted at the stage where the pH is elevated, as explained in (2).

The lowering of pH may be carried out by dialysis and the like, but more preferable from the easiness in operation is to add either an inorganic acid such as hydrochloric acid and sulfuric acid or organic acid such as acetic acid and glycine. As stated above, however, dialysis of the solution is useful particularly when the solution after the refolding still contains a denaturant in the concentration which is high enough to denature a fused heterogenic protein and therefore lowering of the concentration is necessitated. Dialysis achieves the reduction of both pH and the concentration of denaturant simultaneously. The pH should be adjusted to a lower value compared with that of an alkaline solution. The preferable range is 7 - 9, but more preferably 7 - 8, ranging from neutral to weakly alkaline. In this range, from neutral to weakly alkaline, the fused heterogenic proteins can exist and remain stable.

Prior to lowering of pH of the alkaline solution, addition of a sulfhydryl compound such as glutathione in the reduced form, cysteine, 2-mercaptoethanol, dithiothreitol, hydrogen sulfide and the like is recommended in order to prevent disulfide bond from being formed in the fused heterogenic protein and therefore to improve the rate of refolding.

The solubilization treatment step of this invention permits to solubilize insoluble fused heterogenic protein in simple and easy operations. Also the purification treatment step of this invention makes it possible by a simple and easy operation of adding an alkali substance to remove impurities contained in the acid solution containing the fused heterogenic protein which has been obtained by the solubilization treatment of this invention. Further in the refolding treatment of this invention, the pH of the alkaline solution containing the fused heterogenic protein which has been obtained by the solubilization and purification treatments is lowered to obtain the heterogenic protein aimed at in the form in which activity can be expressed. The treatment step according to the present invention is suited to rapidly and conveniently treat even such a large quantity of fused heterogenic protein as not to be treated by previous methods. In particular, when urea is used as protein denaturant, exhaust liquids could be disposed of in a simple operation.

### Detailed Description of the Process according to the present invention:

The present invention will be better understood from the following description of preferred embodiments.

In the following, an example is shown which is related to a fused heterogenic protein composed of NGF and hGH produced by Escherichia coli, to illustrate the present invention in further details. But the scope of this invention is not restricted to the example.

The fused heterogenic protein composed of NGF and hGH (designated hereinafter as NGF-hGH) appearing in the examples below has a molecular weight of about 30,000 dalton. The upperstream region (N terminal side) is a part of hGH composed of 140 amino acids and at the downstream region is located NGF which is composed of 118 amino acids. Since these two portions are separated after the refolding, they are ligated via a specific recognition amino acid sequence (Ile-Glu-Gly-Arg). This NGF-hGH was produced from Escherichia coli as host cells according to the process as disclosed in Japanese Laid-Open Patent Application Sho 61-205485.

### Example 1

After the culture of host cells, 10 g of the bacterial body was suspended in 100 ml of a 20 mM Tris-hydrochloric acid buffer solution (pH = 8) containing 5 % of glycerol and 2 mM of EDTA. The bacterial body was broken into pieces with a ultrasonic cell blender and centrifuged. The NGF-hGH was obtained in the form of precipitate of 8 g together with insoluble fragments of bacterial body. This precipitate was suspended in 200 ml of a 0.1 M aqueous acetic acid solution (pH = 4) containing 7 M of urea. Subsequently a treatment for solubilization was conducted and portion of the precipitate other than the fragments of bacterial body and the like was solubilized.

### Example 2

To the aqueous acetic acid solution containing the NGF-hGH solubilized in Example 1, 200 ml of 10 % by volume of monoethanolamine was added and the pH was adjusted to 12. In the alkaline solution thus produced, protein was precipitated. The precipitate formed was removed by centrifugation together with the impurities which was not solubilized in Example 1.

### Example 3

A 400 ml solution of alkali containing NGF-hGH obtained in Example 2 was dialyzed against 10 l of a 0.15 M Tris-hydrochloric acid buffer solution (pH = 8.5), to simultaneously lower the concentration of urea and the pH to 8.5. No precipitation of protein was observed in this stage of dialysis. After the dialysis, a solution of crude protein containing 500 mg of NGF-hGH was obtained. To a solution containing 1 mg of crude extract of the NGF-hGH obtained was added 10 »g of factor-Xa (produced by Sigma Co.), and the mixture was treated at 37 °C for an hour.

The solution after the treatment was submitted to the electrophoresis on SDS-polyacrylamide to analyze the protein composition. It was confirmed there were present hGH of a molecular weight of about 15,000 dalton and NGF of a molecular weight of about 14,000 dalton. But presence of undigested NGF-hGH was not confirmed which may result owing to an insufficient refolding or a high concentration of a protein denaturant that remained in the solution.

The solution of crude NGF containing NGF obtained as mentioned above was added to a culture solution of rat pheochromocytoma PC12 cells and the activity of NGF was examined. As a result, the activity observed when the NGF solution was added to the culture medium so as to obtain a protein concentration of 1 »g/ml was of the same magnitude as that observable when a control mouse NGF (produced by Sigma Co., 2.5 NGF) was added to result in 100 ng/ml. This fact confirmed that the NGF in the resultant solution was refolded to the state in which the NGF could express the physiological activity.

Meanwhile, the amount of protein in solution was determined by absorbance at 280 nm in this and also subsequent examples.

### Example 4

Following the same procedure as in Example 1, 10 g of a precipitate containing NGF-hGH from host cells was obtained. This precipitate was suspended in 200 ml of a 0.1 M aqueous acetic acid solution (pH = 4) containing 3.5 M of urea, and a solubilization treatment was conducted. Then 200 ml of 10 % by volume of monoethanolamine was added and, after the pH was adjusted to 12, the whole mixture was centrifuged to remove the separated precipitate. Reduced type glutathione was added to result in a concentration of 1 mM, the solution was agitated for 90 min, and the pH of the solution was rendered to 8.5 by adding hydrochloric acid. Thus, a solution was obtained in which NGF was refolded to the state in which the physiological activity could be expressed. This solution contained 450 mg of protein. The activity of NGF was confirmed in the same manner as in Example 3.

### Comparison 1

8 g of the precipitate obtained from host cells in the same manner as in Example 1 were solubilized using 200 ml of an acetic acid solution (pH = 4) containing 3.5 M of urea and dialysed against 10 l of a 0.15 M Tris-hydrochloric acid buffer solution (pH = 8.5) without adding an alkali substance. Most of the protein dissolved in the solution precipitated as precipitate. 50 mg of the precipitate was separated by centrifugation and suspended in the dialysis solution. An attempt to digest NGF and hGH was made using the factor-Xa, but the result proved to be in vain. The electrophoresis on SDS-polyacrylamide evidenced that most of the NGF-hGH remained undigested. Examination of this protein solution on the activity of NGF proved negative. Thus, activity of the NGF was not confirmed.

### Comparison 2

8 g of a precipitate obtained from host cells in a manner similar to that in Example 1 was solubilized with 200 ml of a 10 % by volume solution of monoethanolamine (pH = 12) containing 3.5 M of urea. This solution was dialyzed against 10 l of a 0.15 M Tris-hydrochloric acid solution (pH = 8.5). Then most of the protein dissolved in the solution precipitated as a precipitate. The precipitate was treated in the similar manner as in Comparison 1, but only gave NGF having no activity.

## Claims

1. A process for solubilizing, purifying and refolding a fusion protein consisting of human nerve growth factor and a part of human growth hormone, comprising the following steps:
a) Bringing said fusion protein into contact with an aqueous acid solution of pH 2 to 5 comprising a denaturing concentration of urea,
b) Adjusting the pH to 10 to 13 by adding an alkaline substance and lowering the urea concentration to about 50% of its initial value, thereby precipitating impurities, which are removed, whereas the fusion protein consisting of human nerve growth factor and a part of human growth hormon remains in solution,
c) Lowering the pH of the supernatant to pH 7 to 9, and
d) If necessary, reducing the urea concentration.

2. The process according to claim 1, wherein at least one organic base selected from the group consisting of monoethanolamine, diethylentriamine, triethylenetetramine, and tetraethylenepentamine is used as the alkaline substance.

3. The process according to claim 1, wherein the lowering of pH according to step c, is performed in the presence of a sulfhydryl compound.

4. The process according to claim 1, wherein the aqueous acid solution according to step a) comprises 7 M urea and wherein the urea concentration is lowered to about 3.5 M urea in step b).

## Patentansprüche

1. Verfahren zum Solubilisieren, Reinigen und Falten eines Fusionsproteines, das aus menschlichem Nervenwachstumsfaktor und einem Teil menschlichen Wachstumshormons besteht, umfassend die folgenden Schritte:
(a) das Inkontaktbringen des Fusionsproteines mit einer wäßrigen Säurelösung von pH 2 bis 5, umfassend eine denaturierende Konzentration an Harnstoff,
(b) das Einstellen des pH's auf 10 bis 13 durch Zufügen einer alkalischen Substanz und Erniedrigen der Harnstoffkonzentration auf ungefähr 50 % ihres Anfangswertes, wodurch Unreinheiten ausgefällt werden, die entfernt werden, während das Fusionsprotein, das aus menschlichem Nervenwachstumsfaktor und einem Teil menschlichen Wachstumshormons besteht, in Lösung bleibt,
(c) Erniedrigen des pH's der Überstandes auf pH 7 bis 9, und
(d) Reduzieren der Harnstoffkonzentration, falls notwendig.

2. Verfahren nach Anspruch 1, worin mindestens eine organische Base, ausgewählt aus der Gruppe, die aus Monoethanolamin, Diethylentriamin, Triethylentetramin und Tetraethylenpentamin besteht, als alkalische Substanz verwendet wird.

3. Verfahren nach Anspruch 1, worin das Erniedrigen des pH's gemäß Schritt (c) in Gegenwart einer Sulfhydrylverbindung durchgeführt wird.

4. Verfahren nach Anspruch 1, wobei die wäßrige Säurelösung gemäß Schritt (a) 7 M Harnstoff umfasst, und wobei die Harnstoffkonzentration in Schritt (b) auf ungefähr 3,5 M Harnstoff erniedrigt wird.

## Revendications

1. Procédé de solubilisation, de purification, et de repliage d'une protéine de fusion composée d'un facteur de croissance de nerf humain et d'une partie d'une hormone de croissance humaine comprenant les étapes suivantes :
a) Porter la dite protéine de fusion en contact avec une solution aqueuse acide de pH 2 à 5, comprenant une concentration durée propre à dénaturer,
b) Ajuster le pH entre 10 et 13 en ajoutant une substance alcaline et abaisser la concentration en urée à environ 50 % de sa valeur initiale, faisant précipiter de cette façon les impuretés, qui sont retirées, alors que la protéine de fusion composée d'un facteur de croissance de nerf humain et d'une partie d'une hormone de croissance humaine demeure en solution ,
c) Abaisser le pH du liquide surnageant à un pH compris entre 7 et 9, et
d) Si nécessaire, réduire la concentration en urée .

2. Procédé selon la revendication 1, dans lequel au moins une base organique sélectionnée parmi le groupe consistant en la monoéthanolamine, la diéthylènetriamine, la triéthylènetétramine, et la tétraéthylènepentamine est utilisée comme substance alcaline .

3. Procédé selon la revendication 1, dans lequel l'abaissement de pH selon l'étape (c), est réalisé en présence d'un composé sulfhydrile .

4. Procédé selon la revendication 1, dans lequel la solution aqueuse acide selon l'étape (a) comprend de l'urée 7 M et dans lequel la concentration durée est abaissée à environ 3,5 M durée dans l'étape (b) .
